(19) Europäisches Patentamt
European Patent Office
Office européen des brevets

(11) Publication number : **0 472 494 A2**

## (12) EUROPEAN PATENT APPLICATION

(21) Application number : **91810632.9**

(22) Date of filing : **12.08.91**

(51) Int. Cl.⁵ : **C12N 15/52,** C12N 1/21, C12P 1/04, // A01N63/02, C12N15/78 , (C12N1/21, C12R1:39), (C12P1/04, C12R1:39)

The applicant has filed a statement in accordance with Rule 28 (4) EPC (issue of a sample only to an expert). Accession number(s) of the deposit(s): ATCC 40868, ATCC 40869, ATCC 55171, ATCC 55170, ATCC 55169, ATCC 55175, ATCC 55174 and ATCC 55168.

(30) Priority : **20.08.90 US 570184**
**24.05.91 US 705424**

(43) Date of publication of application :
**26.02.92 Bulletin 92/09**

(84) Designated Contracting States :
**AT BE CH DE DK ES FR GB GR IT LI LU NL SE**

(71) Applicant : **CIBA-GEIGY AG**
**Klybeckstrasse 141**
**CH-4002 Basel (CH)**

(71) Applicant : **U.S. DEPARTMENT OF AGRICULTURE, AGRICULTURAL RESEARCH SERVICE( USDA-ARS)**
**Washington, D.C. 20250 (US)**

(72) Inventor : **Ligon, James M.**
**120 Marquette Drive**
**Cary, NC 27513 (US)**
Inventor : **Hill, Dwight Steven**
**311 Melanie Lane**
**Cary, NC 27511 (US)**
Inventor : **Stein, Jeffrey I.**
**3725 Surry Trail**
**Hillsborough, NC 27278 (US)**
Inventor : **Howell, Charles R.**
**805 Avondale**
**Bryan, Brazos, TX 77802 (US)**
Inventor : **Becker,J. Ole**
**In der Au 1A**
**7854 Inzlingen (DE)**
Inventor : **Lamm, Stephen T.**
**8900 Jeanew Court**
**Raleigh, NC 27613 (US)**

(74) Representative : **Roth, Bernhard M. et al**
**Patentabteilung CIBA-Geigy AG, Postfach**
**CH-4002 Basel (CH)**

(54) **Anti-pathogenic biocontrol agents, genes encoding antibiotics synthesis and the use of said antibiotics.**

(57) Purified bacterial strains that are effective for the inhibition of plant pathogens, including the fungi Rhizoctonia solani and Pythium ultimum have been isolated. These strains are useful as biocontrol agents, and can be used to produce antifungal metabolites, such as antibiotic compounds, active against the plant pathogenic fungi Rhizoctonia solani and Pythium ultimum. Both the purified bacterial strains and the antibiotic compounds can be used as active agents for biocontrol compositions. Genes that encode for one or more enzymes useful in the production of an antibiotic active against the plant pathogenic fungus Rhizoctonia solani and Pythium ultimum have been isolated and used to transform biocontrol agents which are not ordinarily effective against said fungi. The transformed biocontrol agents can be used to control Rhizoctonia solani and Pythium ultimum. Fungicidal compositions comprising the combination of an biological disease controlling Pseudomonas flurescens strain and an acylalanine fungicide are useful for controlling fungi.

EP 0 472 494 A2

RESTRICTION MAP OF pANT CLONES

Fig. 1

## Field of the Invention

The present invention relates to the identification, isolation of novel bacterial strains which are effective biocontrol agents. More specifically, the present invention relates to bacterial strains which have been found to inhibit the growth of fungal plant pathogens. More specifically the present invention relates to previously unknown strains of bacteria, such as members of the genus Pseudomonas, including members of the species Pseudomonas fluorescens, which have been found to produce antifungal metabolits, such as antibiotic compounds, that are effective against fungi, including the plant pathogenic fungi Rhizoctonia solani and Pythium ultimum. The present invention further relates to the identification, isolation, and cloning of genes which encode for the production of said antibiotic substances. More specifically, the present invention relates to genes which encode for substances necessary to the biosynthesis of an antibiotic substance that is effective against the plant pathogenic fungi Rhizoctonia solani and Pythium ultimum. The present invention further relates to a fungicidal composition comprising one or more biological disease controlling Pseudomonas flurescens strains or a biocontrol agent obtainable from said strains and one or more acylalanine fungicides together with an agriculturally acceptable carrier.

It has been recognized that crops grown in some soils are naturally resistant to certain fungal pathogens. Furthermore, soils that are conducive to the development of these diseases can be rendered suppressive, or resistant, by the addition of small quantities of soil from a suppressive field. Scher et al. Phytopathology 70:421 (1980). Conversely, suppressive soils can be made conducive to fungal diseases by autoclaving, indicating that the factors responsible for disease control are biological. Subsequent research has demonstrated that root colonizing bacteria are responsible for this phenomenon known as biological disease control (BDC). Baker et al., Biological control of plant pathogens, (Freeman Press, San Francisco)(1974).

In many cases, the most efficient strains of biological disease controlling bacteria are fluorescent Pseudomonads. Weller et al., Phytopathology, 73:463-469 (1983). These bacteria have also been shown to promote plant growth in the absence of a specific fungal pathogen by the suppression of detrimental rhizosphere microflora present in most soils. Kloepper et al., Phytopathology 71:1020-1024 (1981). Important plant pathogens that have been effectively controlled by seed inoculation with these bacteria include Gaeamannomyces gramminis, the causative agent of take-all in wheat, Cook et al., Soil Biol. Biochem 8:269-273 (1976) and Pythium and Rhizoctonia species, pathogens involved in damping off of cotton. Howell et al., Phytopathology 69:480-482 (1979). Rhizoctonia is a particularly problematic plant pathogen for several reasons. First, it is capable of infecting a wide range of crop plants. Second, there are no commercially available chemical fungicides that are effective in controlling the fungus. Because of these circumstances, an inhibitor against R. solani would be of substantial interest as a potential control for this pathogen.

Many biological disease controlling Pseudomonas strains produce antibiotics that inhibit the growth of fungal pathogens. Howell et al., Phytopathology 69:480-482 (1979); Howell et al. Phytopathology 70:712-715 (1980). These have been implicated in the control of fungal pathogens in the rhizosphere. Several past studies have focused on the effects of mutations that result in the inability of the disease control bacterium or fungus to synthesize these antibiotic compounds. Kloepper et al., Phytopathology 71: 1020- 1024 (1981); Howell et al., Can. J. Microbiol. 29:321-324 (1983). In these cases, the ability of the organism to control the pathogen is reduced, but not eliminated. In particular, Howell et al., Phytopathology 69:480-482 (1979) discloses a strain of Pseudomonas fluorescens which was shown to produce an antibiotic substance that is antagonistic to Rhizoctonia solani.

In Baker et al., Biological Control of Plant Pathogens, (American Phytopathological Society, St. Paul, Minn.)( 1982), pages 61-106, it is reported that an important factor in biological control is the ability of an organism to compete in a given environment. Thus, it is desirable to obtain strains of biocontrol agents which are effective to control the growth of plant pathogens, particularly fungi, such as Rhizoctonia solani and Pythium ultimum and are able to aggressively compete with indigenous bacteria and other microflora that exist in the rhizosphere of the plant.

In order to achieve this objective, it is further desirable to obtain DNA sequences which are useful in conferring resistance to Rhizoctonia solani which may be used to genetically engineer strains of biocontrol agents that combine the ability to control the growth of Rhizoctonia with the ability to control other plant pathogens and/or the ability to aggressively compete in the rhizosphere.

Accordingly, it is one object of the present invention to provide effective fungicide treatments for the protection of crops from plant pathogens, particularly phytopathogenic fungi, such as Oomycetes including Rhizoctonia and Pythium species such as Rhizoctonia solani and Pythium ultimum.

It is another object of the present invention to provide biocontrol strains of bacteria that can be used to control phytopathogenic attack on plants preferably on crop plants.

It is one feature of the present invention to provide novel strains of bacteria especially of the genus Pseudomonas which are useful in producing antifungal metabolites, such as antibiotic compounds, inhibitory to plant pathogens, preferably phytopathogenic fungi, especially Oomycetes including Rhizoctonia and Pythium species such as Rhizoctonia solani and Pythium ultimum. These novel strains represent in purified form biocontrol agents effective against fungal plant pathogens. They produce antibiotics that represent the active principle. These antibiotics can also be used for controlling plant pathogens. Thus, it is a further object of the present invention to provide purified biocontrol agents comprising a fungicidally effective amount of a purified biocontrol agent obtainable from or selected from one of the following Pseudomonas fluorescens strains:

> CGA 266446 (ATCC Accession No. 55171 );
> CGA 266447 (ATCC Accession No. 55170);
> CGA 267356 (ATCC Accession No. 55169);
> CGA 270293 (ATCC Accession No. 55175);
> CGA 270294 (ATCC Accession No. 55174); and
> CGA 281836 (ATCC Accession No. 55168).

It is one advantage of the present invention that biocontrol agents and fungicide treatment for the effective protection of crops against the plant pathogenic fungi Rhizoctonia solani and Pythium ultimum are provided.

It is one further object of the present invention to provide DNA sequences which are useful in coding for the synthesis of antibiotic compounds inhibitory to plant pathogens.

It is yet another object of the present invention to provide genes that can be used to improve the biocontrol capabilities of strains of bacteria used for biocontrol.

It is one feature of the present invention that DNA sequences and genes are provided that aid in the production of an antibiotic substance that is effective against the plant pathogenic fungus Rhizoctonia solani.

It is one advantage of the present invention that biocontrol agents and genes are provided which are active against the plant pathogenic fungus Rhizoctonia solani.

It is another advantage of the present invention that biocontrol agents may be produced which are able to inhibit a broader spectrum of plant pathogens.

It is yet another advantage of the present invention that biocontrol agents may be produced which are able to aggressively compete in the plant rhizosphere, which biocontrol agents contain a DNA sequence that aids in the production of an antibiotic substance that is effective against the plant pathogenic fungus Rhizoctonia solani.

According to the present invention, the above objectives may be carried out by the identification and isolation and use of novel strains of bacterial biocontol agents that are effective against the plant pathogenic fungi Rhizoctonia solani and/or Pythium ultimum and the identification, isolation - preferably from said biocontrol agents - and use of genes that encode for the production of an antibiotic active against the plant pathogenic fungi Rhizoctonia solani and Pythium ultimum. The identification and isolation of such biocontrol agents and genes is important for several reasons. First, R. solani is a particularly pernicious plant pathogen. The affected plants include beans, wheat, tomato and potato, in addition to cotton. Second, there are no environmentally safe and effective fungicide treatments available for the protection of crops from R. solani. Therefore, the use of the biocontol agents including the antibiotic to control or prevent R. solani infections in crop plants by application of a live metabolite or antibiotic-producing bacterium or the antibiotic compound itself, may provide the first environmentaly safe and effective method of control of this pathogen. In addition the bacterium can be used to produce commercial amounts of compounds that are effective for control of plant pathogens, such as R. solani.

In addition, the biocontrol agents of the present invention can be used in mixtures in conjunction with other bacterial strains that otherwise are not effective biocontrol agent for R. solani and P. ultimum and thereby increase the effective range of these biocontrol strains. The use of the biocontrol agents of the present invention in mixtures in order to improve the biocontrol capabilities of other strains of rhizosphre biocontrol agents is also a part of the present invention.

For example, United States Patent No. 4,456,684, (Weller et al.) discloses that take-all, a disease of wheat caused by the fungus Gaeumannomyces gramminis, can be controlled in some cases by the application of bacteria inhibitory to this pathogen to wheat seeds prior to planting. However, where the growth of G.gramminis is effectively under control, R. solani may become a growing problem pathogen of wheat (J. Cook, personal communication). Thus, the biocontrol agents of the present invention can be used together with biocontrol agents intended to protect wheat from take-all and extend their range of effectiveness to include R. soani and P. ultimum.

The bacterial biocontrol agents of the present invention may also be used in combination with chemical compounds, such as chemical fungicides, that are compatible with bacterial strains. Among the preferred chemical fungicides is metalaxyl, which is effective against Pythium ultimum.

The bacterial biocontrol agents of the present invention may also be useful to produce substances which may be used as active ingredients in antifungal compositions for application to the habitat of the phytopathogenic fungi, preferably to the seeds, the leaves or to the soil. The substances produced by the biocontrol agents of the present invention may be used in any manner known in the art, including coating seeds with an effective amount of the substance, or in furrow application of the substance directly into the soil. It is also contemplated that the biocontrol agent may itself be used as the active ingredient in compositions prepared so as not to prove fatal to the biocontrol agent.

We have further demonstrated that the genes which direct the biosynthesis of the antibiotic can be transferred to other bacterial strains, especially pseudomonad strains, that otherwise are not effective biocontrol agents for R. solani and thereby transform them into effective biocontrol strains. The use of the genes to improve the biocontrol capabilities of other strains of rhizosphere biocontrol strains is also part of the present invention. For example, United States Patent No. 4,456,684, (Weller et al.) discloses that take-all, a disease of wheat caused by the fungus Gaemannomyces gramminis, can be controlled in some cases by the application of bacteria inhibitory to this pathogen to wheat seeds prior to planting. However, where the growth of G. gramminis is effectively under control, R. solani may become a growing problem pathogen of wheat (J. Cook, personal communication). The genes for the synthesis of this R. solani-active antibiotic could be introduced into the biocontrol strains currently used to protect wheat from take-all to extend their range of effectiveness to include R. solani.

The recombinant DNA sequences of the present invention may be used to produce an antibiotic substance which may be used as the active ingredient in antifungal compositions for application to soil. The recombinant biocontrol agent of the present invention may be used in any manner known in the art, including coating seeds with an effective amount of the biocontrol agent, or in furrow application of the biocontrol agent directly into the soil. It is also contemplated that the biocontrol agent may itself be used as the active ingredient in compositions prepared so as not to prove fatal to the biocontrol agent.

DEFINITIONS

As used in the present application, the following terms have the meaning set out below:

**Promoter or Regulator DNA sequence:** An untranslated DNA sequence which assists in, enhances, or otherwise affects the transcription, translation or expression of an associated structural DNA sequence which codes for a protein or other DNA product. The promoter DNA sequence is usually located at the 5′ end of a translated DNA sequence, typically between 20 and 100 nucleotides to the 5′ end of the starting site for translation.

**Structural or Coding DNA sequence:** A DNA sequence that is translated or transcribed in an organism to produce an DNA, a protein or other DNA product.

**Associated with/operably linked:** Two DNA sequences which are "associated" or "operably linked" are related physically or functionally. For example, a promoter or regulator DNA sequence is said to be "associated with" a DNA sequence that codes for an RNA or a protein if the two sequences are operably linked, or situated such that the regulator DNA sequence will affect the expression level of the coding or structural DNA sequence.

**Chimeric construct/chimeric DNA sequence:** A recombinant DNA sequence in which a regulator or promoter DNA sequence is associated with, or operably linked to, a DNA sequence that codes for an mRNA or which is expressed as a protein, such that the regulator DNA sequence is able to regulate transcription or expression of the associated DNA sequence. The regulator DNA sequence of the chimeric construct is not normally operably linked to the associated DNA sequence as found in nature. The term "heterologous" is used to indicate a recombinant DNA sequence in which the promoter or regulator DNA sequence and the associated DNA sequence are isolated from organisms of different species or genera.

**Acylalanines:** Fungicidal phenylamide derivatives characterized by the identical mode of action namely by Oomycetes (Phycomycetes) controlling properties. Typical representatives are described in US Patents Nos. 4,742,079, 4, 151,299. 4.046,911 and in Research Disclosure (Aug. 1977) pp40-41 and in Research Disclosure (Nov. 1979) pp 632-633.

**Metalaxyl:** Common name of a typical representative of an acylalanine fungicide having the chemical name N-(2-methoxyacetyl)-N-(2,6-xylyl)-DL-alaninate. Its trade marks are Ridomil, Apron and (Fubol = mixture with mancozeb). Metalxyl is described in US Patent No. 4,151,299.

**Furalaxyl:** Common name of a further typical representative of an acylalanine fungicide having the chemical name N-(2-furoyl)-N-(2,6-xylyl)-DL-alaninate. Its trade mark is Fongarid. Furalaxyl is described in the British Patent No. 1.448,810. It is also described in "The Pesticide Manual" Eight edition (1987) p.6840 published by The British Crop Protection Council.

**Oxadixyl:** Common name of a further typical representative of an acylalanine fungicide having the chemical name 2-Methoxy-N-(2-oxo- 1,3-oxazolidin-3-yl)acet-2′,6′-xylidide. Its trade mark is Sandofan. Oxadixyl is described in the British Patent No. 2,058,059. It is also described in "The Pesticide Manual" Eight edition (1987) p.9175 published by The British Crop Protection Council

**Benalaxyl:** Common name of a further typical representative of an acylalanine fungicide having the chemical name Methyl N-phenylacetyl-N-2,6-xylyl-DL-alaninate. Its trade mark is Galben. Benalaxyl is described in the German Patent No. 2,903,612. It is also described in "The Pesticide Manual" Eight edition (1987) p.660 published by The British Crop Protection Council

**Ofurace:** Common name of a further typical representative of an acylalanine fungicide having the chemical name $\pm$-$\alpha$-2-Cloro-N-2,6-xylyl-y-butyrolactone. Ofurace is described in "The Pesticide Manual" Eight edition (1987) p.9130 published by The British Crop Protection Council.

## Brief Description of the Figures

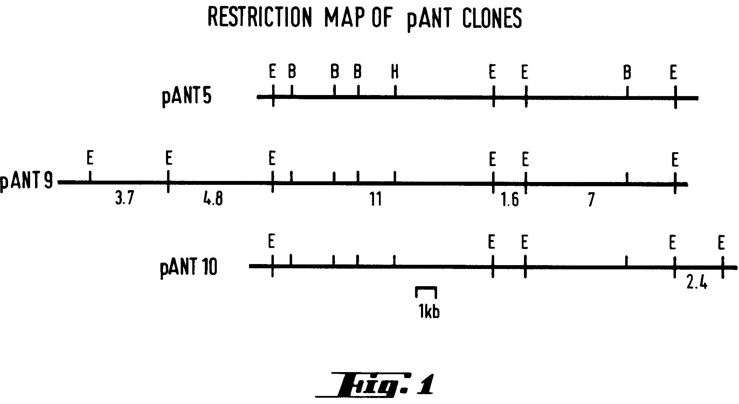

**Figure 1:** This figure shows restriction maps of three cosmid clones, pANT5, pANT9 and pANT10, that were found to complement the ANT⁻ phenotype of mutant 2-1. In this figure, 'B' indicates a BamHI restriction site; 'E' an EcoRI restriction site; and 'H' a HindIII restriction site.

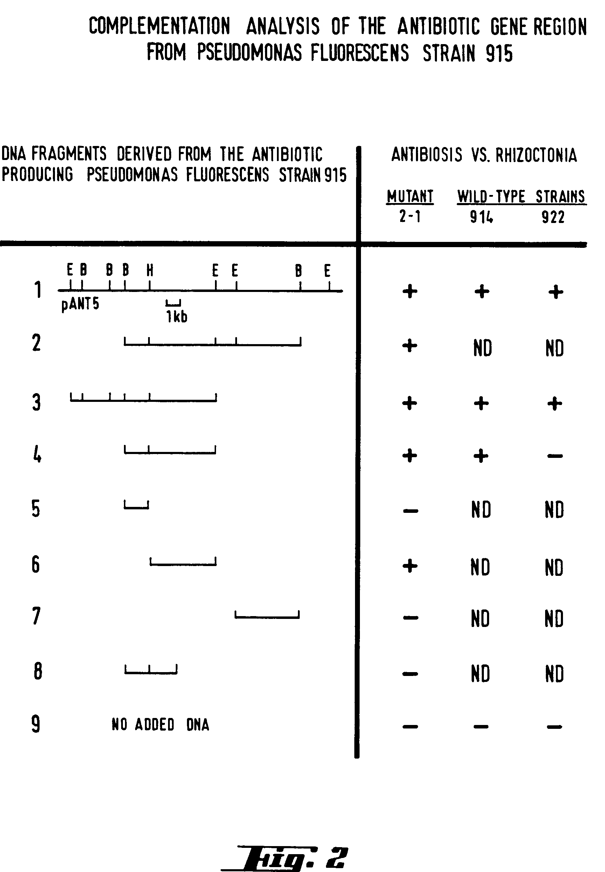

**Figure 2:** This figure shows the ability of DNA subfragments derived from the pANT5 clone of Pseudomonas fluorescens strain 915 to complement the ANT⁻ phenotype of mutant 2-1 and the wild type strains 914 and 922. The subfragment labelled 3 is the approximately 11 kb region which has been called the E11 fragment.

A plus "+" indicates antibiotic production.

A minus "-" indicates no antibiotic production.

"ND" means not determined.

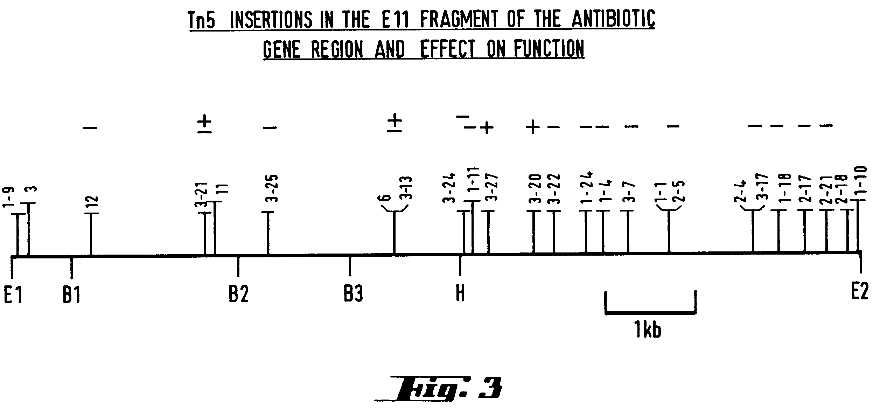

**Figure 3:** This figure indicates the results of Tn5 transposon insertions into the E11 fragment of the antibiotic gene region. Each Tn5 insertion is represented as a vertical line in its approximate position on the genetic map. The numbers above each insertion indicate the identidying number of the insertion. The effect of each insertion on the production of antibiotic in nonproducing wild-type Pseudomonas flurescens strains is indicated above the genetic map in the case of those tested. A minus "-" indicates no antibiotic production, a plus "+" indicates antibiotic production an a plus/minus "$\pm$" indicates variable results.

## Detailed Description of the Invention

In one embodiment of the present invention, purified biocontrol agents are provided which are able to inhibit the growth of fungal pathogens such as Rhizoctonia solani and Pythium ultimum. These biocontrol agents are preferably purified bacterial strains, and more preferably gram negative bacterial strains, such as the pseudomonads. Most preferred as the biocontrol agent are strains of the species Pseudomonas fluorescens. The present invention may also comprise a mixture of bacterial strains, wherein at least one of the bacterial strains is a purified biocontrol agent. The purified biocontrol agents of the present invention may also be conjugated with other bacterial strains for the transfer of their beneficial antibiotic activity.

The biocontrol agents may be applied in any method known for treatment of seed or soil with bacterial strains. For example, see United States Patent No. 4,863,866. These methods may include soil inoculation, seedling inoculation, and seed coating. The strains are effective for biocontrol even if the bacterium is not living. Preferred is, however, the application of the living bacterium.

Another embodiment of the present invention provides methods of inhibiting the growth of phytopathogenic fungi including Oomycetes such as Rhizoctonia solani and Pythium ultimum. In the methods of the present invention, the purified biocontrol agents can be applied in any manner known in the art to be effective for the inhibition of fungal pathogens, such as Rhizoctonia solani and Pythium ultimum, resulting in an effective biocontrol strain.

The present invention embraces antifungal compositions and the preparation thereof in which the active ingredient is either the antibiotic substance produced by the recombinant biocontrol agent of the present invention (details, see below) or is the purified biocontrol bacterial strain itself. The present invention embraces antifungal compositions and the preparation thereof in which the active ingredient is the antifungal metabolite or antibiotic compound produced by the purified biocontrol bacterial strain of the present invention.

Where the active ingredient is a biocontrol bacterial strain, the biocontrol preparation may be applied in any manner known for seed and soil treatment with bacterial strains. The bacterial strain may be homogeneously mixed with one or more compounds or groups of compounds described herein, provided such compound is compatible with bacterial strains. Preferred compounds are acylalanine fungicides and agriculturally acceptable carriers. The present invention also relates to methods of treating plants, preferably crop plants, which comprise application of the bacterial strain or the antibiotic substance produced by the strain, or fungicidal compositions containing the bacterial strain or the antibiotic substance produced by the strain, to the plants.

The active ingredient of the present invention may also be an antifungal metabolite, such as an antibiotic compound, produces by the biocontrol agents of the present invention. The present invention als relates to methods of treating plants, which comprise application of the antifungal metabolite, such as an antibiotic compound, or antifungal compostions containing the metabolite, to plants.

The active ingredients of the present invention including said metabolites are normally applied in the form of compositions and can be applied to the crop area or plant to be treated, simultaneously or in succession, with other compounds. These compounds can be both fertilizers or micronutrient donors or other preparations that influence plant growth. They can also be selective herbicides, insecticides, fungicides (preferably acylalanine fungicides), bactericides, nematicides, mollusicides or mixtures of several of these preparations, if desired, together with further agriculturally acceptable carriers, surfactants or application-promoting adjuvants customarily employed in the art of formulation. Suitable carriers and adjuvants can be solid or liquid and correspond to the substances ordinarily employed in formulation technology, e.g. natural or regenerated mineral substances, solvents, dispersants, wetting agents, tackifiers, binders or fertilizers.

Preferred methods of applying an active ingredient of the present invention or an agrochemical composition of the present invention which contains at least one of the antifungal metabolites produced by the bacterial strains of the present invention are leaf application, seed coating and soil application. The number of applications and the rate of application depend on the intensity of infestation by the corresponding pathogen (type of fungus). However, the active ingredients can also penetrate the plant through thr roots via the soil (systemic action) by impregnating the locus of the plant with a liquid composition, or by applying the compounds in solid form to the soil, e.g. in granular form (soil application). The active ingredients may also be applied to seeds (coating) by impregnating the seeds either with a liquid formulation containing active ingredients, or coating them with a solid formulation. In special cases, further types of application are also possible, for example, selective treatment of the plant stems or buds.

Furthermore, it has surprisingly been found that the combined application of one or more biological disease controlling Pseudomonas flurescens strains or of a purified biocontrol agent obtainable from Pseudomonas flurescens strains together with one or more acylalanine fungicides exhibits a significantly enhanced activity against plant pathogens of the order Oomycetes (Phycomycetes) including Phythium and Rhizoctonia species.

The activity achieved by sai combinations is decisively greater than the activity to be expected by adding together the activities of the components individually (synergism).

Thus, the present invention further relates to a fungicidal composition comprising one or more biological disease controlling Pseudomonas flurescens strains or a biocontrol agent obtainable from said strains and one or more acylalanine fungicides together with an agriculturally acceptable carrier.

Favourable mixing ratios of the two active principles Pseudomonas flurescens strain (= I) and the acylalanine fungicide (= II) are: I:II = from 100:1 to 1:100, preferably from 10:1 to 1:10. Enhanced activity is already achieved when the chemical fungicide is used in normal concentrations and the bacterial strain is added in amounts of from 50 g to 5 kg a.i. per hectare.

Thus, the term 'biocontrol agent' used throughout the present specification and the claims encompasses purified biological disease controlling Pseudomonas flurescens strains alone or in combination with one or more acylalanine fungicides. IT further encompasses the active principle including antifungal metabolits, such as antibiotic compounds alone or in combination with one or more acylalanine fungicides. Preferred are in this context the Pseudomonas flurescens strains CGA 266446, CGA 266447, CGA 267356, CGA 270293, CGA 270294 and CGA 281836. Preferred acylalanine fungicides are metalaxyl, furalaxyl, oxadixyl, benalaxyl and ofurace. Especially preferred is the combination with metalaxyl.

The active ingredients are used in unmodified form or, preferably, together with a suitable agriculturally acceptable carrier. Such carriers are adjuvants conventionally employed in the art of agricultural formulation, and are therefore formulated in known manner to emulsifiable concentrates, coatable pastes, directly spryable or dilutable solutions, dilute emulsions, wettable powders, soluble powders, dusts, granulates, and also encapsulations, for example, in polymer substances. Like the nature of the compositions, the methods of application, such as spraying, atomizing, dusting, scattering or pouring, are chosen in accordance with the intended objected and the prevailing circumstances. Advantageous rates of application are normally from 50 g to 5 kg of active ingredient (a.i.) per hectare ("ha", approximately 2.471 acres), preferably from 100 g to 2 kg a.i./ha, most preferably from 200 g to 500 g a.i./ha.

The formulations, compositions or preparations containing the active ingredients and, where appropriate, a solid or liquid adjuvant, are prepared in known manner, for example by homogeneously mixing and/or grinding the active ingredients with extenders, for example solvents, solid carriers and, where appropriate, surface-active compounds (surfactants).

7

Suitable solvents for compositions including those that contain the antifungal metabolites produced by the biocontrol bacterial strains of the present invention include aromatic hydrocarbons, preferably the fractions having 8 to 12 carbon atoms, for example, xylene mixtures or substitued naphthalenes, phthalates such as dibutyl phthalate or dioctyl phthalate, aliphatic hydrocarbons such as cyclohexane or paraffins, alcohols and glycols and their ethers and esters, such as ethanol, ehtylene glycol monomethyl or monethyl ether, ketones such as cyclohexanone, strongly polar solvents such as N-methyl-2-pyrrolidone, dimethyl sulfoxide or dimethyl formamide, as well as epoxidized vegetable oils such as epoxidized coconut oil or soybean oil; or water.

The solid carriers used e.g. for dusts and dispersible powders, are normally natural mineral fillers such as calcite, talcum, kaolin, montmorillonite or attapulgite. In order to improve the physical properties it is also possible to add highly dispersed silicic acid or highly dispersed absorbent polymers. Suitable granulated adsorptive carriers are porous types, for example pumice, broken brick, sepiolite or bentonite; and suitable nonsorbent carriers are materials such as calcite or sand. In addition, a great number of pregranulated materials of inorganic or organic nature can be used, e.g. especially dolomite or pulverized plant residues.

Depending on the nature of the active ingredient to be used in the formulation, sutiable surface-active compounds are nonionic, cationic and/or anionic surfactants having good emulsifying, dispersing and wetting properties. The term "surfactants" will also be understood as comprising mixtures of surfactants.

Suitable anionic surfactants can be both water-soluble soaps and water-soluble synthetic surface-active compounds.

Suitable soaps are the alkali metal salts, alkaline earth metal salts or unsubstituted or substituted ammonium salts of higher fatty acids (chains of 10 to 22 carbon atoms), for example the sodium or potassium salts of oleic or stearic acid, or of natural fatty acid mixtures which can be obtained for example from coconut oil or tallow oil. The fatty acid methyltaurin salts may be used.

More frequently, however, so-called synthetic surfactants are used, especially fatty sulfonates, fatty sulfates, sulfonated benzimidazole derivatives or alkylarylsulfonates.

The fatty sulfonates or sulfates are usually in the form of alkali metal salts, alkaline earth metal salts or unsubstituted or substituted ammonium salts and have a 8 to 22 carbon alkyl radical which also includes the alkyl moiety of alkyl radicals, for example, the sodium or calcium salt of lignonsulfonic acid, of dodecylsulfate or of a mixture of fatty alcohol sulfates obtained from natural fatty acids. These compounds also comprise the salts of sulfuric acid esters and sulfonic acids of fatty alcohol/ethylene oxide adducts. The sulfonated benzimidazole derivatives preferably contain 2 sulfonic acid groups and one fatty acid radical containing 8 to 22 carbon atoms. Examples of alkylarylsulfonates are the sodium, calcium or triethanolamine salts of dodecylbenzenesulfonic acid, dibutylnaphthalenesulfonic acid, or of anaphthalenesulfonic acid/formaldehyde condensation product. Also suitable are corresponding phosphates, e.g. salts are preferably in the form of halides, methylsulfates or ethylsulfates, e.g. stearyltrimethylammonium chloride or benzyldi(2-chloroethyl)ethylammonium bromide or salts of the phosphoric acid ester of an adduct of p-nonylphenol with 4 to 14 moles of ethylene oxide.

Non-ionic surfactants are preferably polyglycol ether derivatives of aliphatic or cycloaliphatic alcohols, or saturated or unsaturated fatty acids and alkylphenols, said derivatives containing 3 to 30 glycol ether groups and 8 to 20 carbon atoms in the (aliphatic) hydrocarbon moiety and 6 to 18 carbon atoms in the alkyl moiety of the alkylphenols.

Further suitable non-ionic surfactants are the water-soluble adducts of polyethylene oxide with polypropylene glycol, ethylenediamine propylene glycol and alkylpolypropylene glycol containing 1 to 10 carbon atoms in the alkyl chain, which adducts contain 20 to 250 ethylene glycol ether groups and 10 to 100 propylene glycol ether groups. These compounds usually contain 1 to 5 ethylene glycol units per propylene glycol unit.

Representative examples of non-ionic surfactants re nonylphenolpolyethoxyethanols, castor oil polyglycol ethers, polypropylene/polyethylene oxide adducts, tributylphenoxypolyethoxyethanol, polyethylene glycol and octylphenoxyethoxyethanol. Fatty acid esters of polyoxyethylene sorbitan and polyoxyethylene sorbitan trioleate are also suitable non-ionic surfactant.

Cationic surfactants are preferably quaternary ammonium salts which have, as N-substituent, at least one $C_8$-$C_{22}$alkyl radical and, as further substituents, lower unsubstituted or halogenated alkyl, benzyl or lower hydroxyalkyl radicals. The salts are preferably in the form of halides, methylsulfates or ethylsulfates, e.g. stearyltrimethylammonium chloride or benzyldi(2-chloroethyl)ethylammonium bromide.

The surfactants customarily employed in the art of formulation are described, for example, in "McCutcheon's Detergent and Emulsifiers Annual," MC Publishing Corp. Ringwood, New Jersey, 1979, and Sisely and Wood, "Encyclopedia of Surface Active Agents," Chemical Publishing Co., Inc. New York, 1980.

The surfactants customarily employed in the art of formulation are described, for example, in "McCutcheon's Detergents and Emulsifiers Annual," MC Publishing Corp. Ringwood, New Jersey, 1979, and Sisely and Wood, "Encyclopedia of Surface Active Agents," Chemical Publishing Co., Inc. New York, 1980.

The agrochemical compositions usually contain from about 0.1 to about 99 %, preferably about 0.1 to about 95 %, and most preferably from about 3 to about 90 % of the active ingredient, from about 1 to about 99.9 %, preferably from about 1 to about 99 %, and most preferably from about 5 to about 95 % or a solid or liquid adjuvant, and from about 0 to about 25 %, preferably about 0.1 to about 25 %, and most preferably from about 0.1 to about 20 % of a surfactant.

Whereas commercial products are preferably formulated as concentrates, the end user will normally employ dilute formulations.

A fragment of DNA containing genes encoding traits required for the biosynthesis of an antibiotic that is effective against the plant pathogenic fungus Rhizoctonia solani has been cloned and characterized. The DNA fragment was derived from Pseudomonas fluorescens strain 915. This strain was isolated from the roots of a cotton plant grown in a Texas cotton field and was identified as an effective biocontrol strain of Pythium ultimum-and Rhizoctonia solani-induced damping off of cotton.

The DNA fragment containing these genes has been transferred to two different P. fluorescens strains that are not known to produce an antibiotic effective against R. solani and are not effective biocontrol agents for R. solani-induced damping-off in cotton. In each case, the P. fluorescens strains harboring the antibiotic gene fragment demonstrated antibiotic production in vitro based on inhibition of R. solani growth on agar plates. In addition, the strains were tested in the greenhouse and were demonstrated to be effective biocontrol agents for R. solani-induced damping off in cotton.

In one embodiment of the present invention, recombinant DNA sequences are obtained which comprise a structural DNA sequence which codes for the production of an antibiotic substance which inhibits the growth of the fungus Rhizoctonia solani. This DNA sequence may be derived from biocontrol agents which are effective to inhibit the growth of Rhizoctonia. Preferably the DNA sequence may be derived from the clone pANT5, which was isolated from a strain of Pseudomonas fluorescens. More preferably, the DNA sequence may comprise the approximately 11 kb E11 fragment of pANT5. The clone pANT5 has been deposited with ATCC and has been designated ATCC accession number 40868. A plasmid containing the 11 kb E11 fragment of pANT5 has been deposited with ATCC and has been designated ATCC accession number 40869.

The recombinant DNA sequences of the present invention may be chimeric and may be heterologous or homologous. The recombinant DNA sequences of the present invention may further comprise one or more regulatory DNA sequences operably linked to the structural DNA sequence above. Such regulatory DNA sequences include promoter sequences, leader sequences, and other DNA sequences which may affect the expression of the regulatory DNA sequences, as well as those fragments of a regulator DNA sequence that are able to act with such effect.

In another embodiment of the present invention, biocontrol agents are provided which are able to inhibit the growth of Rhizoctonia solani. These biocontrol agents may be bacteria, plant cells or animal cells, but are preferably bacterial strains, and more preferably gram negative bacterial strains, such as the pseudomonads. Most preferred as the biocontrol agent are strains of the genus Pseudomonas fluorescens.

Another embodiment of the present invention provides methods of inhibiting the growth of the fungus Rhizoctonia solani. In the methods of the present invention, the recombinant DNA sequences coding for inhibition of the pathogen is introduced into the genome of a biocontrol agent, such as a bacterial strain which is not ordinarily effective as an inhibitor of the pathogen Rhizoctonia solani, resulting in an effective biocontrol strain.

DNA in the form of plasmids can be transferred from one bacterium to another by a sexual process termed conjugation. Plasmids capable of conjugal transfer contain genes that code for the synthesis of sex pili. Sex pili are hollow tubes that join the plasmid-containing bacterium (the donor) with another bacterium (the recipient) and through which replicated copies of the plasmid pass from the donor to the recipient. This procedure occurs naturally in nature and is utilized in the laboratory as a method of transferring genes from one bacterium to another. For some strains of bacteria, such as Pseudomonas, conjugal transfer of DNA is the preferred method since these bacteria are not readily transformed with extraneous DNA.

In yet another embodiment of the present invention, methods are provided for obtaining an antibiotic substance which is effective to inhibit the growth of the fungus Rhizoctonia solani. This method comprises introducing the recombinant DNA sequences of the present invention into the genome of a biocontrol agent to form a transformed biocontrol agent, allowing the transformed biocontrol agent to produce an antibiotic substance, and extracting the antibiotic substance from the transformed biocontrol agent.

While the present invention is described below with reference to specific embodiments thereof, it will be appreciated that numerous variations, modifications, and embodiments are possible, and accordingly, all such variations, modifications and embodiments are to be regarded as being within the spirit and scope of the present invention.

## Examples

### Example 1: Collection of plant and soil samples.

Plant and soil samples are collected from active cotton-growing areas in Texas. The plant samples are removed directly from the field approximately three to four weeks after planting. Plant samples are placed in plastic bags and stored in an ice chest uni returned to the lab. Soil samples are placed in five-gallon plastic containers with lids and stored at 25°C. Where it is not possible to obtain fresh plant samples, soil from the fields is planted with cotton seed at the lab and incubated in the growth chamber to obtain seedlings for sampling purposes.

### Example 2: Preliminary screening of samples for antibiotic activity.

Dilution series are made from the root systems and adhering soil of cotton seedlings down to $10^{-7}$, and 1 ml aliquots are mixed with 9 mls of molton agar of the following media: trypticase-soy agar, nutrient agar, potato-dextrose agar, King's medium B agar, and soil extract agar. Washed segments of the seedling root systems are also plated directly onto agar plates.

After a three to four day incubation period the plates are inoculated with Rhizoctonia solani infested dry-ground wheat bran or agar plugs of Pythium ultimum. The plates are observed periodically for the presence of clear zones in the fungal growth around bacterial colonies. Those colonies showing inhibitory or antibiotic activity are streak-purified and placed in dual culture with the fungal pathogens to confirm their antibiotic activity.

The putative antagonists thus identified are graded on a relative scale as to their antibiotic activity against R. solani and P. ultimum on nutrient agar (high nutrient) and soil extract agar (low nutrient). Strains demonstrating good inhibitory activity on one or more media are stored in 0.85 % saline at 5°C and in sterile soil cultures to maintain their genetic purity.

### Example 3: Further screening of novel isolates

Inhibitory strains are examined on the basis of morphological, physiological and antibiotic characteristics. Those strains found not to be duplicates of other isolates are streaked from saline solution onto nutrient agar plates and incubated for three days. The bacteria are then wahed from three plates with 30 mls of steril water.

Inoculum of R. solani is prepared by growing the fungus in moist sterile wheat bran or millet for approximately one to two weeks, air drying the material for several days, then grinding the inoculm to 20 mesh with a Wiley mill.

Inoculum of P. ultimum is prepared by growing the fungus on sterile moist millet seed for one week. The material is then air-dried and broken up into individual seeds. Alternatively, inoculum of P. ultimum may be grown in still liquid cultures of cholesterol-V8 juice medium for approximately 10 days. teh mycelial mats are wahed and macerated in a waring blender to obtain only oospores.

A suspension of propagules may be mixed into soil at the rate of 2,000 oospores per gram of soil, and the soil incubated for approximately 6 weeks before use, in order to overcome the dormancy factor associated with fresh oospores.

Soil used in the tests for biocontrol efficacy is Lufkin Fine Sandy Loam with a pH of 5.7. The soil is passed through a 1/4 inch screen and adjusted to a 15 % moisture level. The soil is infested with the pathogens by mixing inoculum and soil together in a cement mixer for 20 minutes at the rate of approximately 0. 15 to 0.4 gms of inoculum/13.6 kg of soil for R. solani and 20.4 gms of inoculum/13.6 kg of soil for P. ultimum. This material may optimally be used for approximately 3 weeks before preparing fresh material.

Infested soil is placed in 20 oz. plastic drinking cups containing 2″ x 10″ cotton cheesecloth wicks passed through a 5/8″ hole in the bottom. The wick extends to the soil surface. Five holes 2 cm deep are poked into the soil surface in each cup around the wick, and a seed of the cotton variety Stoneville 213 is placed in each hole.

Alternatively, soil infested with R. solani is placed in flats containing about 13.6 kg of soil and partitioned into four sections. Four rows of ten seed each are planted in each section, each seed is treated with about 0.5 ml of bacterial suspension on the seed and about 0.5 ml on the soil surface and the flats are incubated at 25°C with a 12 hours photoperiod for approximately 14 days. Each section is watered with approximately 250 ml of deionized water every other day. At the end of that period, counts are made of emerged and surviving seedlings.

Soil infested with P. ultimum oospores may be placed in 5 gm lots into test tubes containing about 0.75 ml of water or bacterial suspension, and one cotton seed is planted into the center of each. Ten tubes are treated with each bacterial strain, and the tubes are incubated at about 15°C in the dark for about 7 days. The tubes are then moved to 25°C and a 12 hours photoperiod for about 3 to 5 days. Counts are made of emerged and surviving seedlings. Bacterial strains whose treatment results in 50 % or better seedling survival as an average of three tests are stored in 0.375 ml nutrient broth and 0. 125 ml glycerol at -70°C. Cultures of these strains, identified to species, are subjected to further testing.

Treatment of the seed and soil consists of placing 0.5 ml of bacterial suspension from the selected strains on each seed, covering the seed with infested soil, then dispersing 3 ml of bacterial suspension over the entire soil surface in the cup. The soil surface is then sprayed lightly with water, and the cup is covered with a single layer of 0.5 ml plastic wrap and secured with a rubber band. Controls are treated with water.

Treated and control cups are placed in a growth chamber with a 20°C 12 hours photoperiod and a 16°C dark period. After approximately four days without supplementary water, the plastic wrap is removed and the wick is placed in a water reservoir 2.5 inches below the cup bottom. After a further six-day incubation, counts are made of emerged and surviving seedlings. Survivors are removed from the soil and examined for evidence of disease or phytotoxic effects on the hypocotyl and root system.

Those strains whose treatment results in 65 % or more healthy seedlings are retained, and identified where possible by species according to the morphological and physiological descriptions provided in Bergey's Manual of Determinative Bacteriology as well as with the use of identifying tests, such as Rapid NFT (from API Analytab Products). This test measures a number of key biochemical traits that are used to classify the bacteria. This particular kit is designed to distinguish Gram negative, oxidase positive, bacteria, of which, Pseudomonas is one.

## Example 4: Identification of effective biocontrol strains

a) Samples in a given area are taken on the basis of different soil types, different cotton varieties grown, and variations in previous cropping history. Samples are ideally taken when the cotton plant is at the seeling stage, but can be taken at a later stage.

In the first screening, 22 plant and soil samples from five different growing areas are obtained. From dilution assay plates made from these samples in accordance with Example 2. A total of 114 bacteria and 20 actinomycetes are isolated which produce compounds active against R. solani or P. ultimum.

Of the total numbers of bacteria, 103 strains are active against R. solani on nutrient agar, while 59 strains are active on soil extract agar. Of the latter group, eight are also active against R. solani on soil extract agar, but not on nutrient agar. This indicates that approximately 50 % of the total number of isolated bacterial strains are active only on nutrient agar, while the other 50 % are active on both media.

Of those isolates showing activity against P. ultimum, 79 are active on nutrient agar and 53 on soil exract agar. Within these two groups 39 are active only on nutrient agar, nine are active only on soil exract agar, and 84 are actie on both media.

b) In a second screening, a total of 135 isolates are taken from the sample dilution plates and tested in vitro for activity against R. solani and P. ultimum. Of these, 35 are active against both pathogens.

A total of 230 strains are assayed in the growth chamber for efficacy as biocontrol agents of Pythium or Rhizoctonia induced damping-off of cotton seedlings. Of these isolates, 29 give better than 65 % seedling survival in R. solani infested soil, and 12 give better than 65 % survival in P. ultimum invested soil. A number of these strains are deemed to be duplicates of one another on the basis of antibiotic and biocontrol tests and other physiological characterization. Six selected pseudomonad strains are advanced for further study, along with 10 antibiotic producing actinomycetes. All pseudomonad strains are identified as belonging to Pseudomonas fluorescens.

c) In a third screening, a total of 359 isolates screened in dilution plates are retained and genetically stabilized for assay in the growth chamber for biocontrol efficacy. 158 of these isolates are fully tested for biocontrol of Rhizoctonia solani or Pythium ultimum. Of these, 13 demonstrate good biocontrol of seedling disease incited by R. solani or P. ultimum, or both. These isolates are identified to species, stored under glycerol at -70°C, and advanced for further study.

## Example 5: Cultivation of bacteria and fungi for screening assays

a) Cultivation of bacteria

The bacterial strains are stored in 50 % glycerol at -80°C prior to use. One loop from the stored culture is suspended in 5 ml Luria Broth (LB: 10 g Bacto-Tryptone, Difco; 5 g yeast extract, Oxoid; 0.25 g $MgSO_4H_2O$; 8 g NaCl; and 1 l distilled water; pH 7) and shaken at 150 rpm and 25°C over night. 100 ml LB is inoculated with 1 ml of the pre-culture and incubated under the same conditions. 10 ml of the last culture are centrifuged (10 min. at 10,000 rpm), and the pellet is resuspended in 200 ml saline (0.8 % NaCl) giving a concentration of approximately $10^8$ cfu/ml.

For exact determination a dilution series ($10^0$ to $10^{-8}$, 20 ul in 180 ul) is prepared in a microtiter plate and drops of 10 ul are spotted onto Luria Agar (LB with 1.5 % Bacto-Agar, Difco) with an Eppendorf pipette. The cfu are counted after 24 hours incubation at 28°C.

b) Cultivation of Rhizoctonia solani

Rhizoctonia solani is grown on Potato Dextrose Agar (PDA, Difco), pH 5.6 in a petri dish. A 300 ml-Erlenmeyer flask with 25 g millet and 50 ml distiled water each was autoclaved and incubated with one agar plug (5 mm diameter) from a PDA culture of R. solani. After incubation at 20°C in the dark for 3 weeks the overgrown millet is air-dried and ground in a Culatti mill (1 mm sieve, 6000 rpm).

c) Cultivation of Pythium ultiumum

Pythium ultimum is grown on Malt Agar (Oxoid), pH 5.6 in a petri dish. One agar plug (6 mm diameter) from this culture is transferred to a petri dish with 8 ml oatmeal agar (150 g Oatmeal 3 ml 1.5 % chloesterin in ethanol, and 1 l distilled water), with a slant surface. Two hours later 13 ml of sterile distiled water are added, and the plates are incubated for 10 to 14 days in the dark. The mycelium which grow from the agar surface into the water is transferred to a mixer and cut into small pieces. The concentration of oospores is counted in a Thoma chamber and adjusted with distilled water to $2 \times 10^4$/ml.

**Example 6: Performance of screening assays**

**a) Pathosystem R. solani - cotton**

Plastic pots (280 ml) are filled first with a thick bottom layer (1 cm) of Vemex F and then with a mixture (7:3 v/v) of steamed, but recolonized St. Aubin soil (sieved, 3 mm) and Vermex F. Previously surface sterilized cotton seeds (Cossypium hirsutum, cv. Delta Pine) are incubated in hand-warm water for 30 min. and seeded 1 to 1.5 cm deep into the soil. 5 mg of millet powder infested with R. solani (see 2.1.2) is added to the center of the pot 1 cm below the surface. All pots are watered with 60 ml water each

Two hours later each pot is drenched with 20 ml of a bacterial suspension ( $10^8$ cfu/ml, see 2.1.1) of 20 ml Monceren (1 mg WP 5/ml) resp. resulting in approximately $7 \times 10^6$ cfu/ml soil or 20 ppm Monceren resp. All pots are randomized and incubated in a growth chamber set to 23°C at daytime (14 hours) and 18°C at night (10 hours) and 50 % rel. humidity. The soil is kept moist.

The emergence is recorded after 5, 9 and 13 days. After 20 days the disease incidence is evaluated by rating the plant either healthy ("1") or not germinated or diseased (both "0"), if the root or hypocotyl appears brown an rotted. For each pot with 5 seeds the ratings are summarized resulting in a number of "0" (all plants diseased or not germinated), "1", "2", "3", "4" or "5" (all 5 plants healthy). The numbers from 5 pots per treatment are averaged. The biocontrol efficacy of each treatment in an experiment is given in % in comparison to the control with no treatment and no pathogen (NT.NP, defined as 100 % biocontrol) and the control with pathogen only (NT.P, defined as 0 % biocontrol).

The biocontrol efficacy of six strains tested against R. solani in cotton is presented in Table 1. Strains CGA 267356 and CGA 281836 show a high level of control (80 % and 76 %, respectively). The other four strains are somewhat less active but nevertheless exhibit excellent biocontrol activity.

**b) Pathosystem P. ultimum - cotton**

Soil and seeds are prepared as described in part a) above, and pots are watered with 50 ml distilled water each. After 1 hour. 20 ml of an oospore suspension ($2 \times 10^4$/ml, see 2.1.3) are drenched into the soil (approximately 1400 cfu/cm³). After another two hours 20 ml of a bacterial suspension ($10^8$ cfu/ml, see 2. 1.1) or 20 ml of Ridomil (WP 25, 1 mg/ml) resp. is added to each pot resulting in approximately $7 \times 10^6$ cfu/cm³ soil or 20 ppm Ridomil resp. The pots are incubated under the same conditions as described above, and mergence and disease incidence are recorded as mentioned earlier (see part a) above).

Table 2 shows the biocontrol efficacy of the same strains against P. ultimum. Strain CGA 266446 proves to be the most effective strain (56 % biocontrol). The rest of the strains show lesser beneficial effects (30 to 44 % biocontrol).

### c) Pathosystem R. solani - tomato

Bacteria ( 120 ml/6 kg) are added to steamed Pro-mix BX in a mechanical tumbler and tumbled for 5 minutes. The bacterized Pro-mix is allowed to stand in loosely covered buckets for three days before the pathogen inoculum (0.02 % colonized oat grains) is added. The mixture is then tumbled for five additional minutes and placed in pots. 5 pots are seeded with 10 tomato seed (cv. Bonny Best) for each treatment. Terraclor (wp 75, 10.3 k ai/ha) is added as a control treatment without bacterial treatment and is added as a drench immediately after planting. Each treatment is replicated 4 times. The pots are kept in the headhouse for the first 4 days after seeding (~23°C) and then moved to the green-house for the remainder of the trial. After 15 days the number of healthy plants are counted. A treatment with pathogen but with no bacterial control treatment is prepared as a pathogen check treatment.

Table 3 shows the results of trials of four rhizobacterial strains. CGA 266446 and CGA 267356 significantly reduce the disease. CGA 266447 and CGA 270294 are somewhat less active but also demonstrate beneficial effects.

### d) Pathosystem R. solani - cucumber

Soil is prepared by mixing bacterial cells into raw commercial potting soil at the rate of 1 ml of cells at a density of approximately $10^9$ cells/ml per 10 g of soil. Concurrently, powdered Rhizoctonia solani-infested ground millet seed is mixed into the soil at a rate of 10 ml/10 g of soil. The soil is loaded into commercial seedling flats, each consisting of 12 small pots 3 cm square that hold approximately 10 g of soil. A single cucumber seed is planted in each of the 12 pots in each seedling flat and three flats are prepared for each treatment. Uninfested controls that have no pathogen or bacteria added to the soil, and infested controls that have pathogen but no bacteria, are included in each experiment. The flats are placed in a growth chamber in which a constant temperature of 28°C is maintained and are kept moist by watering twice daily from an overhead sprinkler. At 12 days after planting, the number of surviving plants in each treatment is recorded.

Table 4 shows the results of trials of 3 rhizobacterial strains. CGA 267356 and CGA 270293 significantly reduce the disease. CGA 26647 is less effective.

### Example 7: Isolation and efficacy of antipathogenic strains

### a) Pseudomonas fluorescens strain CGA 266446

Strain CGA 266446 is identified as belonging to the species Pseudomonas fluorescens. This strain is isolated from the rhizosphere of a mature cotton plant in sandy clay loam in Burleson Co., Texas. The previous crop was corn. The strain is active against both Rhizoctonia solani and Pythium ultimum on nutrient agar but has only low activity on soil extract agar. Treatment of cotton in soil infested with R. solani results in 71 % disease control (Table 1). Treatment of cotton in soil infested with P. ultimum results in 56 % disease control (Table 2). Treatment of tomato in soil infested with R. solani results in 123 % disease control (Table 3).

### b) Pseudomonas fluorescens strain CGA 266447

Strain CGA 266447 is identified as belonging to the species Pseudomonas fluorescens. This strain is isolated from the same area as, and has similar activity to, Strain CGA 266446. Treatment of cotton in soil infested with R. solani results in 70 % disease control (Table 1). Treatment of cotton in soil infested with P. ultimum results in 30 % disease control (Table 2). Treatment of tomato in soil infested with R. solani results in 54 % disease control (Table 3). Treatment of cucumber in soil infested with R. solani results in 10 % disease control (Table 4).

### c) Pseudomomas fluorescens strain CGA 267356

Strain CGA 267356 is identified as belonging to the species Pseudomonas fluorescens. This strain is isolated from the rhizosphere of a cotton variety "GP3774" in blackland soil from a farm near Aquilla, Hill Gounty, Texas. The previous crop was wheat. The strain is active against Rhizoctonia solani on both nutrient agar and soil extract agar. It is active against Pythium ultimum on nutrient agar only. Treatment of cotton in soil infeseted with R. solani results in 80 % disease control (Tabel 1). Treatment of cotton in soil infested with P. ultimum results in 44 % disease control (Table 2). Treatment of tomato in soil infested with R. solani results in 131 % disease control (Table 3). The strain produces an antibiotic which is effective to treat R. solani and P. ultimum, which is described in patent application serial number 570, 184, filed on August 20, 1990, the specification of which is hereby incorporated by reference. Treatment of cucumber in soil infested with R. solani results in 67 % disease control (Table 4).

### d) Pseudomonas fluorescens strain CGA 270293

Strain CGA 270293 is identified as belonging to the species Pseudomonas fluorescens. This strain is isolated from the rhizosphere of a cotton variety "Stoneville 213 " in Norwood silty loam from the Texas A&M Research Farm, in Brazos County, Texas. The previous crop was cotton. The strain is active against Rhizoctonia solani on both nutrient agar and soil extract agar. It is active against Pythium ultimum on nutrient agar only. Treatment of cotton in soil infested with R. solani results in 68 % disease control (Table 1). Treatment of cotton in soil infested with P. ultimum results in 33 % disease control (Tabel 2). Treatment of cucumber in soil infested with R. solani results in 53 % disease control (Table 4).

### e) Pseudomonas fluorescens strain CGA 270294

Strain CGA 270294 is identified as belonging to Pseudomonas fluorescens. This strain was isolated from the rhizosphere of cotton variety "Stoneville 213 " in Miles fine sandy loam from Chilicothe, Texas. The previous crop was cotton variety "Paymaster 145". This strain was active against R. solani on nutrient agar ans soil extract agar, and aganist P. ultimum on nutrient agar. The strain is active against both R. solani and P. ultimum in infested soil. Treatment of cotton results in 40 % disease control in soil infested with R. solani (Table 1) and 34 % disease control in soil infested with P. ultimum (Tabel 2). Treatment of tomato in soil infesetd with R. solani results in 46 % disease control (Table 3).

### f) Pseudomonas fluorescens strain CGA 281836

Upon further culturing of a sample of strain CGA 266447, it is found that two distinct strains of bacterium are present. The second bacterium, denoted strain CGA 281836, is morphologically distinct from CGA 266447 and exhibits good biocontrol activity. CGA 281836 is identified as belonging to the species Pseudomonas fluorescens. Treatment of cotton in soil infested with R. solani results in 76 % disease control (Table 1). Treatment of cotton in soil infested with P. ultimum results in 39 % disease control (Table 2).

### Example 8: Inhibition of Rhizoctonia solani

The active antifungal metabolite can be extracted from the growth medium of bacterial strains that produce inhibitory antibiotics. For example, using strain CGA 267356, this is accomplished by extraction of the growth medium with 80 % acetone followed by removal of the acetone by evaporation and a second extraction with diethyl ether. The diethyl ether is removed by evaporation and the dried extract is resuspended in a small volume of water. Small aliquots of the antibiotic extract applied to small sterile filter paper discs placed on an agar plate will inhibit the growth of R. solani, indicating the presence of the active antibiotic compound. Since the antibiotic is readily extractable with organic solvents, it is likely to have cyclic or aromatic groups in its structure as is common of many antibiotics known to be produced by other pseudomonads.

### Example 9: Synergistic combination of P. fluorescences with the acylalanine fungicide mealaxyl to control damping-off of cotton in soil infested with Rhizoctonia solani and Pythium ultimum

Strains CGA 281836, CGA 267356 and CGA 270293 are each applied to non-sterile soil as a drench at $2 \times 10^8$ cfu/ml soil, while metalaxyl fungicide is either drenched (Ridomil, at 0.02, 0.5 or 2 ppm) or coated onto seeds (Apron, at 35 g a.i.(100 kg seed). P. ultimum is introduced as an oospore suspension (1400 spores/ml soil). R. solani is introduced as a pelleted millet powder (5 mg in the center of each pot). After incubation for 19 days in the greenhous, the hypocotyls of cotton seedings are rated for disease on an observation scale.

Almost complete control of damping-off is achieved when strain CGA 267356 is applied together with Ridomil at 2 ppm. Using Apron instead of Ridomil results in the same level of control. Strain CGA 267356 alone still gives significant suppression of both pathogens. Metalaxyl fungicide alone fails to control the disease complex.

0.02 ppm Ridomil alone gives 40 % suppression of P. ultimum. The combination of 0.02 ppm Ridomil with Strain CGA 267356 increases the level of control to more than 60 %. Strain CGA 281836 gives results essentially as good as Strain CGA 267356. Strain CGA 270293 does not significantly suppress the seedling disease

EP 0 472 494 A2

complex.

The above demonstrates that combined application of biocontrol bacterial strains with a reduced rate of metalaxyl fungicide achieves almost complete control of the seedling disease complex in cotton caused by R. solani and P. ultimum.

## Example 1O: Antibiotic Gene Isolation

The wild-type Pseudomonas fluorescens strain 11c-1-38 (strain 915) is mutated by exposure to the mutagen N-Methyl-N'-nitro-N- nitrosoguanidine. Approximately two dozen antibiotic-affected mutants are identified by screening individual mutants for their ability to inhibit the growth of P. ultimum and R. solani on nutrient agar. Most of these are shown to have reduced, or no, antibiotic activity against one of the two phytopathogenic fungi, but are not affected in their inhibition of the other fungus.

However, one mutant, that we have named mutant 2-1, is found to lack antibiotic activity against both test fungi. These results strongly indicate that P. fluorescens strain 11c-1-38 produces two distinct antibiotics, one effective against P. ultimum and the other effective against R. solani, rather than one antibiotic that is effective against both fungi.

A gene library of total DNA isolated from the parent strain was constructed by partial SalI restriction of the DNA, size fractionation to yield fragment of 20-30 kilobases, and ligation into XhoI-restricted vector pVK100. Knauf et al., Plasmid 8:45-54 (1982). The gene library is transferred to the antibiotic mutant 2-1 by triparental conjugation with E. coli harboring the tra+ plasmid pRK2013. Ditta et al., Proc. Natl. Acad. Sci. USA 77:7347-7351 (1980). Transgenic exconjugants are tested for the production of antibiotic by measurinng growth inhibition of P. ultimum and R. solani. The overlapping clones are identified that restored antibiotic activity against R. solani, but not against P. ultimum, to mutant 2- 1. Restriction maps of these clones are determined and are shown in Figure 1.

## Example 11: Characterization of the antibiotic gene region

The genetic region necessary for the functional complementation of antibiotic biosynthesis in mutant 2-1 is defined by subcloning portions of the larger region and assessing their ability to complement mutant 2-1 for antibiosis (Figure 2). The smallest fragment that is demonstrated to complement the mutation is the 4.9 kb HindIII/EcoRI fragment, which is indicated as subfragment 6 in Figure 2 and is designated subfragment H/E 4.9. Some of the subcloned DNA fragments derived from the antibiotic gene parent clone, which has been designated pANT-5, are transferred to two wild type P. fluorescens strains, 11c-1-33 (strain 914) and 11-1-6 (strain 922), that were not ordinarily able to produce antibiotic against R. solani.

The results, shown in Figure 2, indicated that an 11 kb EcoRI subfragment, which is indicated as subfragment 3 in Figure 2 and is designated subfragment E11, imparts R. solani-active antibiosis to both strains. In order to better define the genetic bounaries of this phenomenon, transposon mapping is used to obtain a functional map of the antibiotic gene region.

Twenty-five independent transposon Tn5 insertions are generated in the 11 kb EcoRI fragment (Figure 3). The ability of most of these to impart R. solani-active antibiosis to the two test strains is determined and the results are depicted in Figure 3. The results indicate that most of the 4.9 kb HindIII/EcoRI fragment on the right side of the 11 kb EcoRI fragment is involved in antibiotic synthesis as are regions to the left of the HindIII site. However, two Tn5 insertions in a short region immediately to the right of the HindIII site has no effect on the synthesis of the antibiotic. This indicates that this region contains DNA that is not necessary for antibiosis.

## Example 12: Inhibition of Rhizoctonia solani

The active antibiotic compound can be extracted from the growth medium of the transformed P. fluorescens strain that produces this antibiotic. This is accomplished by extraction of the medium with 80 % acetone followed by removal of the acetone by evaporation and a second extraction with diethyl ether. The diethyl ether is removed by evaporation and the dried extract is resuspended in a small volume of water. Small aliquots of the antibiotic extract applied to small sterile filter paper discs placed on an agar plate will inhibit the growth of R. solani, indicating the presence of the active antibiotic compound. Since the antibiotic is readily extractable with organic solvents, it is likely to have cyclic or aromatic groups in its structure as is common of many antibiotics known to be produced by other pseudomonads.

### Example 13: Formulations of antifungal compositions employing liquid compositions

In the following examples, percentages of composition are given by weight and the active ingredient* is either

a) a transformed Pseudomonas fluorescens bacteria producing the antibiotic substance inhibitory to the target pathogen or

b) an antifungal metabolit produced by Pseudomonas fluorescens bacteria which are inhibitory to the target pathogen or

c) an 1:1 combination of the Pseudomonas flurescens strains CGA 266446, CGA 266447, CGA 267356, CGA 270293, CGA 270294 or CGA 281836 and metalaxyl.

## 1. Emulsifiable concentrates:

| | a | b | c |
|---|---|---|---|
| Active ingredient* | 20 % | 40 % | 50 % |
| Calcium dodecylbenzenesulfonate | 5 % | 8 % | 6 % |
| Castor oil polyethlene glycol ether (36 moles of ethylene oxide) | 5 % | - | - |
| Tributylphenol polyethylene glycol ether (30 moles of ethylene oxide) | - | 12 % | 4 % |
| Cyclohexanone | - | 15 % | 20 % |
| Xylene mixture | 70 % | 25 % | 20 % |

Emulsions of any required concentration can be produced from such concentrates by dilution with water.

## 2. Solutions:

| | a | b | c | d |
|---|---|---|---|---|
| Active ingredient* | 80 % | 10 % | 5 % | 95 % |
| Ethylene glycol monomethyl ether | 20 % | - | - | - |
| Polyethylene glycol 400 | - | 70 % | - | - |
| N-methyl-2-pyrrolidone | - | 20 % | - | - |
| Epoxidised coconut oil | - | - | 1 % | 5 % |
| Petroleum distillate (boiling range 160-190°) | - | - | 94 % | - |

These solutions are suitable for application in the form of microdrops.

## 3. Granulates:

| | a | b |
|---|---|---|
| Active ingredient* | 5 % | 10 % |
| Kaolin | 94 % | - |
| Highly dispersed silicic acid | 1 % | - |
| Attapulgite | - | 90 % |

The active ingredient is dissolved in methylene chloride, the solution is sprayed onto the carrier, and the solvent is subsequently evaporated off in vacuo.

| 4. Dusts: | a | b |
|---|---|---|
| Active ingredient* | 2 % | 5 % |
| Highly dispersed silicic acid | 1 % | 5 % |
| Talcum | 97 % | - |
| Kaolin | - | 90 % |

Ready-to-use dusts are obtained by intimately mixing the carriers with the active ingredient.

### Example 14: Formulation of antifungal compositions employing solid compositions

In the following examples, percentages of compositions are by weight and the active ingredient* can be either one or more of the transformed Pseudomonas fluorescens bacteria producing the antibiotic substance inhibitory to the target pathogen or one or more of the antifungal metabolits produced by Pseudomonas fluorescens bacteria which are inhibitory to the target pathogen:

| 1. Wettable powders: | a | b | c |
|---|---|---|---|
| Active ingredient* | 20 % | 60 % | 75 % |
| Sodium lignosulfonate | 5 % | 5 % | - |
| Sodium lauryl sulfate | 3 % | - | 5 % |
| Sodium diisobutylnaphthalene-sulfonate | - | 6 % | 10 % |
| Octylphenol polyethylene glycol ether (7-8 moles of ethylene oxide) | - | 2 % | - |
| Highly dispersed silicic acid | 5 % | 27 % | 10 % |
| Kaolin | 67 % | - | - |

The active ingredient is thoroughly mixed with the adjuvants and the mixture is thoroughly ground in a suitable mill, affording wettable powders which can be diluted with water to give suspensions of the desired concentrations.

### 2. Emulsifiable concentrate:

| | |
|---|---|
| Active ingredient* | 10 % |
| Octylphenol polyethylene glycol ether (4-5 moles of ethylene oxide) | 3 % |
| Calcium dodecylbenzenesulfonate | 3 % |
| Castor oil polyglycol ether | 4 % (36 moles of ethylene oxide) |
| Cyclohexanone | 30 % |
| Xylene mixture | 50 % |

Emulsions of any required concentration can be obtained from this concentrate by dilution with water.

### 3. Dusts:

| | a | b |
|---|---|---|
| Active ingredient* | 5 % | 8 % |
| Talcum | 95 % | - |
| Kaolin | - | 92 % |

Ready-to-use dusts are obtained by mixing the active ingredient with the carriers, and grinding the mixture in a suitable mill.

### 4. Extruder granulate:

| | |
|---|---|
| Active ingredient* | 10 % |
| Sodium lignosulfonate | 2 % |
| Carboxymethylcellulose | 1 % |
| Kaolin | 87 % |

The active ingredient is mixed and ground with the adjuvants, and the mixture is subsequently moistened with water. The mixture is extruded and then dried in a stream of air

### 5. Coated granulate:

| | |
|---|---|
| Active ingredient* | 3 % |
| Polyethylene glycol 200 | 3 % |
| Kaolin | 94 % |

The finely pound active ingredient is uniformly applied, in a mixer, to the kaolin moistened with polyethylene glycol. Non-dusty coated granulates are obtained in this manner.

## 6. Suspension concentrate:

| | |
|---|---|
| Active ingredient* | 40 % |
| Ethylene glycol | 10 % |
| Nonylphenol polyethylene glycol (15 moles of ethylene oxide) | 6 % |
| Sodium lignosulfonate | 10 % |
| Carboxymethylcellulose | 1 % |
| 37 % aqueous formaldehyde solution | 0.2 % |
| Silicone oil in 75 % aqueous emulsion | 0.8 % |
| Water | 32 % |

The finely ground active ingredient is intimately mixed with the adjuvants, giving a suspension concentrate from which suspensions of any desire concentration can be obtained by dilution with water.

### Example 15: Field trial of biocontrol strains

Strains of biocontrol strains are stored in 50 % glycerol at -80°C. One loop from the stored culture is suspended in 5 ml Luria Broth (LB: 10 g Bacto-Tryptone, Difco; 5 g yeast extract, Oxold; 0.25 g $MgSO_4xH_2O$; 8 g NaCl; and 11 distilled water; pH7) and shaken at 150 rpm and 25°C for 24 hours. 100 ml LB is inoculated with 1 ml of the preculture and incubated under the same conditions. After 16 hours, the culture is centrifuged for 10 minutes at 10,000 rpm, and the pellet is resuspended in saline (0.8 % NaCl) and adjusted to $3x10^9$ cfu/ml (OD2). Thus, 100 ml of culture will give approximately 200-300 ml drench of OD2.

A hemocytometer and/or spectrophotometer is used to adjust the concentration of bacteria in the drench. Otherwise, a standard salt solution of a known OD (e.g., Phillips' Milk of Magnesia™ = $Mg(OH)_2$) can be used to adjust the OD of the drench. If a centrifuge is not available, the whole culture broth has to be applied; a hemocytometer is then used to determine the cfu/ml.

For exact determination, a dilution series ($10^0$ to $10^{-8}$; 20 ul in 180 ul) is prepared in a microtiter plate and drops of 10 ul are spotted onto Luria Agar (LB with 1.5 % Bacto-Agar, Difco) with an Eppendorf pipette. The cfu are counted after 24 hours incubation at 28°C.

250 ml of the bacterial suspension per 10'(=1rep) are drenched onto the covered seeds (200 seeds per rep). A handheld sprayer or watering can free of pesticide residues is used to apply the drench in a narrow band or approximately 1.5 inches with.

Rhizoctonia solani and Pythium ultimum are prepared for inoculation as in Example 5 above.

Emergence is recorded at 10 days after planting to assess pre-emergence damping off. Stand are recorded at 21 days and 28 days after planting to assess post-emergence damping-off.

## Table 1

Biocontrol activity in % of the strains used in the screening against <u>Rhizoctonia</u> <u>solani</u> in cotton plants.

| Strain | Mean Percent Biocontrol Activity[1] |
|---|---|
| CGA 266446 | 71 |
| CGA 266447 | 70 |
| CGA 267356 | 80 |
| CGA 270293 | 68 |
| CGA 270294 | 40 |
| CGA 281836 | 76 |

1 =  Uninfested control designated 100 % biocontrol  Infested control designated 0 % biocontrol

## Table 2

Biocontrol activity in % of the strains used in the screening against <u>Pythium</u> <u>ultimum</u> in cotton plants.

| Strain | Mean Percent Biocontrol Activity[1] |
|---|---|
| CGA 266446 | 56 |
| CGA 266447 | 30 |
| CGA 267356 | 44 |
| CGA 270293 | 33 |
| CGA 270294 | 34 |
| CGA 281836 | 39 |

1 =  Uninfested control designated 100 % biocontrol Infested control designated 0 % biocontrol.

## Table 3

Biocontrol activity in % of the strains used in the screening against <u>Rhizoctonia</u> <u>solani</u> in tomato plants.

| Treatment | Stand 15 DAP[a] | | | | Mean | % Biocontrol Activity[1] |
|---|---|---|---|---|---|---|
| | Rep1 | Rep2 | Rep3 | Rep4 | | |
| NP.NT[b] | 9 | 10 | 5 | 10 | 8.50 | 100 |
| P.NT[c] | 5 | 4 | 6 | 6 | 5.25 | 0 |
| Terraclor | 10 | 10 | 9 | 10 | 9.75 | 138 |
| CGA 266446 | 9 | 9 | 10 | 9 | 9.25 | 123 |
| CGA 266447 | 7 | 5 | 6 | 10 | 7.00 | 54 |
| CGA 267356 | 9 | 10 | 10 | 9 | 9.50 | 131 |
| CGA 270294 | 7 | 6 | 6 | 7 | 6.75 | 46 |

a = Days After Planting

b = No Pathogen, No Treatment (Uninfested control)

c = Pathogen, No Treatment (Infested control)

1 = Uninfested cotnrol designated 100 % biocontrol
Infested control designated 0 % biocontrol

## Table 4

Biocontrol activity in % of the strains used in the screening against <u>Rhizoctonia solani</u> in tomato plants.

|  | Stand 12 DAP[a] | | | | |
|---|---|---|---|---|---|
| Treatment | Rep1 | Rep2 | Rep3 | Mean | % Biocontrol Activity[1] |
| NP.NT[b] | 12 | 11 | 12 | 11.67 | 100 |
| P.NT[c] | 7 | 10 | 9 | 8.67 | 0 |
| CGA 266447 | 9 | 9 | 9 | 9.00 | 10 |
| CGA 267356 | 10 | 11 | 11 | 10.67 | 67 |
| CGA 270293 | 9 | 11 | 11 | 10.33 | 53 |

a = Days After Planting

b = No Pathogen, No Treatment (Uninfested control)

c = Pathogen, No Treatment (Infested control)

1 = Uninfested cotnrol designated 100 % biocontrol

Infested control designated 0 % biocontrol

### DEPOSITS under Budapest Treaty with the American Type Culture Collection

The clone pANT5 has been deposited one August 17, 1990 with ATCC and has been designated ATCC accession number 40868. A plasmid containing the 11 kb E11fragment of pANT5 has been deposited at the same date and has been designated ATCC accession number 40869.

The following <u>Pseudomonas fluorescens</u> strains have been deposited by the applicant on April 24, 1991 with the American Type Culture Collection, 12301 Parklawn Drive, Rockville, Md. 20852, in accordance with the Budapest Treaty:

CGA 266446 (ATCC Accession No. 55171);

CGA 266447 (ATCC Accession No. 55170);

CGA 267356 (ATCC Accession No. 55169);

CGA 270293 (ATCC Accession No. 55175);

CGA 270294 (ATCC Accession No. 55174); and

CGA 281836 (ATCC Accession No. 55168).

## Claims

1. A purified biocontrol agent effective against fungal pathogens, comprising a fungicidally effective amount of a purified biocontrol agent obtainable from or selected from one of the following <u>Pseudomonas fluorescens</u> strains:

CGA 266446 (ATCC Accession No. 55171);

CGA 266447 (ATCC Accession No. 55170);

CGA 267356 (ATCC Accession No. 55169);

CGA 270293 (ATCC Accession No. 55175);

CGA 270294 (ATCC Accession No. 55174); and

CGA 281836 (ATCC Accession No. 55168).

EP 0 472 494 A2

2.  A purified biocontrol agent effective against fungal pathogens according to claim 1, wherein the fungal pathogenes are selected from the group consisting of Rhizoctonia solani and Pythium ultimum.

3.  Fungicidal composition comprising one or more biological disease controlling Pseudomonas flurescens strains or a biocontrol agent obtainable from said strains and one or more acylalanine fungicides together with an agriculturally acceptable carrier.

4.  Composition according to claim 3, wherein the Pseudomonas flurescens strain is selected from the group consisting of the Pseudomonas flurescens strains:
    CGA 266446 (ATCC Accession No. 55171);
    CGA 266447 (ATCC Accession No. 55170);
    CGA 267356 (ATCC Accession No. 55169);
    CGA 270293 (ATCC Accession No. 55175);
    CGA 270294 (ATCC Accession No. 55174); and
    CGA 281836 (ATCC Accession No. 55168).

5.  Composition according to any one of claims 3 or 4, wherein the acylalanine fungicide s selected fro the group consisting of metalaxyl, furalaxyl, oxadixyl, benalaxyl and ofurace.

6.  Composition according to any one of claims 3 to 5, wherein the biocontrol agent is that of claim 1 or claim 2.

7.  A method of controlling a phytopathogenic fungi comprising introducing a fungicidally effective amount of a biocontrol agent of any one of claims 1 to 2 or of a fungicidal composition of any one of claims 3 to 6 into the environment where the phytopathogenic fungus is to be inhibited.

8.  A method of obtaining antibiotic substance which effectively inhibits the growth of a fungal pathogen comprising culturing a purified biocontrol agent of claim 1 under conditions sufficient to produce an antibiotic substance; and extracting the antibiotic substance by commen means.

9.  A recombinant DNA sequence which codes for one or more enzymes necessary for the synthesis of an antibiotic substance, wherein said antibiotic substance inhibits the growth of the fungus Rhizoctonia solani.

10. The recombinant DNA sequence of claim 9, wherein said DNA sequence is obtainable from the clone pANT5.

11. The recombinant DNA sequence of claim 10, wherein said DNA sequence comprises the approximately 11 kb E11 fragment.

12. A biocontrol agent comprising a recombinant DNA sequence selected from the group consisting of the DNA sequences as claimed in any one of claims 9 to 11.

13. The biocontrol agent of claim 12, wherein the biocontrol agent is a bacterial strain.

14. The biocontrol agent of claim 13, wherein the bacterial strain is a pseudomonad.

15. The biocontrol agent of claim 14, wherein the bacterial strain is from the genus Pseudomonas fluorescens.

16. A method of inhibibng the growth of the fungus Rhizoctonia solani comprising:
    a) introducing the recombinant DNA sequence of any one of claims 9 to 11 into the genome of a biocontrol agent to form a transformed biocontrol agent;
    b) applying the transformed biocontrol agent into the environment where the fungus Rhizoctonia solani is to be inhibited.

17. A method of obtaining an antibiotic substance which is effective to inhibit the growth of the fungus Rhizoctonia solani comprising:
    a) introducing the recombinant DNA sequence of any one of claims 9 to 11 into the genome of a biocontrol agent to form a transformed biocontrol agent;

b) allowing the transformed biocontrol agent to produce an antibiotic substance; and

c) extracting the antibiotic substance from the transformed biocontrol agent by commen means.

18. A Pseudomonas flurescens selected from the group consisting of the strains:

CGA 266446 (ATCC Accession No. 55171 );
CGA 266447 (ATCC Accession No. 55170);
CGA 267356 (ATCC Accession No. 55169);
CGA 270293 (ATCC Accession No. 55175);
CGA 270294 (ATCC Accession No. 55174); and
CGA 281836 (ATCC Accession No. 55168).

**Claims for the following Contracting State: ES**

1. Fungicidal composition comprising one or more biological disease controlling Pseudomonas flurescens strains or a biocontrol agent obtainable from said strains and one or more acylalanine fungicides together with an agriculturally acceptable carrier.

2. Composition according to claim 1, wherein the Pseudomonas flurescens strain is selected from the group consisting of the Pseudomonas flurescens strains: CGA 266446 (ATCC Accession No. 55171 ); CGA 266447 (ATCC Accession No. 55170); CGA 267356 (ATCC Accession No. 55169); CGA 270293 (ATCC Accession No. 55175); CGA 270294 (ATCC Accession No. 55174); and CGA 281836 (ATCC Accession No. 55168).

3. Composition according to any one of claims 1 or 2, wherein the acylalanine fungicide s selected fro the group consisting of metalaxyl, furalaxyl, oxadixyl, benalaxyl and ofurace.

4. Composition according to any one of claims 1 to 3, wherein the bioconaol agent is a purified biocontrol agent effective against fungal pathogens, comprising a fungicidally effective amount of a purified biocontrol agent obtainable from one of the following Pseudomonas fluorescens strains: CGA 266446 (ATCC Accession No. 55171); CGA 266447 (ATCC Accession No. 55170); CGA 267356 (ATCC Accession No. 55169); CGA 270293 (ATCC Accession No. 55175); CGA 270294 (ATCC Accession No. 55174); and CGA 281836 (ATCC Accession No. 55168).

5. Composition according to any one of claim 1 to 4, wherein the fungal pathogenes are selected from the group consisting of Rhizoctonia solani and Pythium ultimum.

6. A method of controlling a phytopathogenic fungi comprising introducing a fungicidally effetive amount either of a biocontrol agent obtainable from a biological disease controlling Pseudomonas flurescens strain or of a fungicidal composition of any one of claims 3 to 6 into the environment where the phytopathogenic fungus is to be inhibited.

7. A method of obtaining an antibiotic substance which effectively inhibits the growth of a fungal pathogen comprising culturing a purified biocontrol agent of claim 1 under conditions sufficient to produce an antibiotic substance; and extracting the antibiotic substance by commen means.

8. A method of inhibiting the growth of the fungus Rhizoctonia solani comprising:
a) introducing a recombinant DNA sequence which codes for one or more enzymes necessary for the production of an antibiotic substance, wherein said antibiotic substance inhibits the growth of the fungus Rhizoctonia solani into the genome of a biocontrol agent to form a transformed biocontrol agent;
b) applying the transformed biocontrol agent into the environment where the fungus Rhizoctonia solani is to be inhibited.

9. A method of obtaining an antibiotic substance which is effective to inhibit the growth of the fungus Rhizoctonia solani comprising:
a) introducing a recombinant DNA sequence which codes for one or more enzymes necessary for the production of an antibiotic substance, wherein said antibiotic substance inhibits the growth of the fungus Rhizoctonia solani into the genome of a biocontrol agent to form a transformed biocontrol agent;
b) allowing the transformed biocontrol agent to produce an antibiotic substance; and

c) extracting the antibiotic substance from the transformed biocontrol agent by commen means.

10. A method for controlling phytopathogenic Oomycetes comprising applying an fungicidally effective amount of a bacterial strain selected from the group consisting of the Pseudomonas flurescens strains: CGA 266446 (ATCC Accession No. 55171); CGA 266447 (ATCC Accession No. 55170); CGA 267356 (ATCC Accession No. 55169); CGA 270293 (ATCC Accession No. 55175); CGA 270294 (ATCC Accession No. 55174); and CGA 281836 (ATCC Accession No. 55168) alone or in combination with one or more acylalanine fungicides and optionally together with an agriculturally acceptable carrier to the fungus or its habitat.

# RESTRICTION MAP OF pANT CLONES

Fig. 1

# COMPLEMENTATION ANALYSIS OF THE ANTIBIOTIC GENE REGION
## FROM PSEUDOMONAS FLUORESCENS STRAIN 915

| DNA FRAGMENTS DERIVED FROM THE ANTIBIOTIC PRODUCING PSEUDOMONAS FLUORESCENS STRAIN 915 | ANTIBIOSIS VS. RHIZOCTONIA | | |
|---|---|---|---|
| | MUTANT 2-1 | WILD-TYPE STRAINS 914 | 922 |
| 1 | + | + | + |
| 2 | + | ND | ND |
| 3 | + | + | + |
| 4 | + | + | − |
| 5 | − | ND | ND |
| 6 | + | ND | ND |
| 7 | − | ND | ND |
| 8 | − | ND | ND |
| 9  NO ADDED DNA | − | − | − |

*Fig. 2*

Tn5 INSERTIONS IN THE E11 FRAGMENT OF THE ANTIBIOTIC
GENE REGION AND EFFECT ON FUNCTION

Fig. 3